# EUROPEAN PATENT APPLICATION

(11) **EP 1 586 651 A1**
(43) Date of publication of application: **19.10.2005**
(21) Application number: 04008748.8
(22) Date of filing: 13.04.2004
(51) Int. Cl.: C12N 15/82, A01H 5/00

(54) **Means and method for modifying the biomass of plants**

(71) Applicant: Max-Planck-Gesellschaft zur Förderung der Wissenschaften e.V., 80539 München (DE)
(72) Inventor: Weigel, Detlef, Prof. Dr., 72076 Tübingen (DE); Schwab, Rebecca, 72072 Tübingen (DE)
(74) Representative: Wibbelmann, Jobst

(57) **Abstract**

The present invention relates to means and methods for modifying biomass yield and/or plant growth and/or plant architecture of plants. In particular, it concerns transgenic plants exhibiting an increased biomass yield and plant growth rate compared to the corresponding wild-type plants. The plants according to the present invention are characterized by containing altered levels of a microRNA, in particular microRNA that targets members of the *SPL* family of genes encoding SPL transcription factors.

## Description

The present invention relates to means and methods for modifying biomass yield and/or plant growth and/or plant architecture of plants. In particular, it concerns transgenic plants exhibiting an increased biomass yield and plant growth rate compared to the corresponding wild-type plants.

There is a growing interest in the production of plant structures having traits considered desirable by the agricultural or breeding industry. In particular plants wherein the economically useful parts form a large proportion of the modified plants without negatively affecting the health of the plants would be of agricultural, commercial and technical value. To date, there have been only few attempts to modify plant traits, in particular the key trait biomass, by genetic engineering approaches. WO 00/56905 for example discloses a method for promoting or modifying plant growth, yield and architecture in plants comprising the increased expression of at least two cell cycle interacting proteins, more particularly a protein kinase and a cyclin. US 2003/0172402 A1 discloses a method for improving growth, biomass production and xylem fiber length in trees by over-expressing a key regulatory gene in the biosynthesis of the plant hormone gibberellin.

Recently, small RNAs of about 20-24 nudeotides, so-called MicroRNAs (miRNAs), have emerged as important regulators of gene expression. MicroRNAs have been found across both animal and plant kingdoms in great numbers. They have been shown to originate from fold-back RNA precursors, which are processed by the ribonuclease DICER in animals and DICER-LIKE in plants. Plants like *Arabidopsis thaliana* or animals like zebra fish with mutations in DICER or DICER-LIKE show pleiotropic phenotypes, including lethality for null alleles, suggesting that miRNAs play a very important role in the development of multicellular organisms.

The fold-back structures of plant miRNAs are included in transcripts that are developmentally regulated, may contain introns and be polyadenylated, and are most likely transcribed by RNA polymerase II, thus sharing many features with protein-coding messenger RNAs (Aukerman & Sakai, Plant Cell *15*, 2730-2741; Schmid *et al,* Development *130,* 6001-6012).

The mechanisms described for miRNA action require interaction with target transcripts through complementary base pairing, leading to either translational repression or cleavage of the transcript. In animals, miRNAs bind to motives in the 3'UTR of the transcripts. The resulting RNA-RNA interaction, which includes several non-pairing bases, leads to reduced protein accumulation, presumably through attenuation of translation. Compared to animals, target sequences for plant miRNAs are mostly found in the coding regions of the target mRNAs and often share a higher degree of complementary to the miRNAs. Both mechanisms - cleavage and repression of translation - are found to regulate target gene expression in plants. Generally, miRNA targets have turned out to be key regulators of developmental pathways and most of them belong to various classes of transcription factors. As their targets, miRNA precursor genes often belong to families with several loci in the genome that give rise to the same, or very similar, mature miRNA sequences. This mature sequence is in most cases perfectly conserved even in distantly related species, such as between the dicotyledonous plant *Arabidopsis thaliana* and the monocotyledonous plant rice. Even non-seed plants, such as mosses, share perfectly conserved miRNAs with flowering plants, which demonstrates the general applicability of specific miRNAs to any species of higher plants (Floyd & Bowman, Nature 428, 485-486).

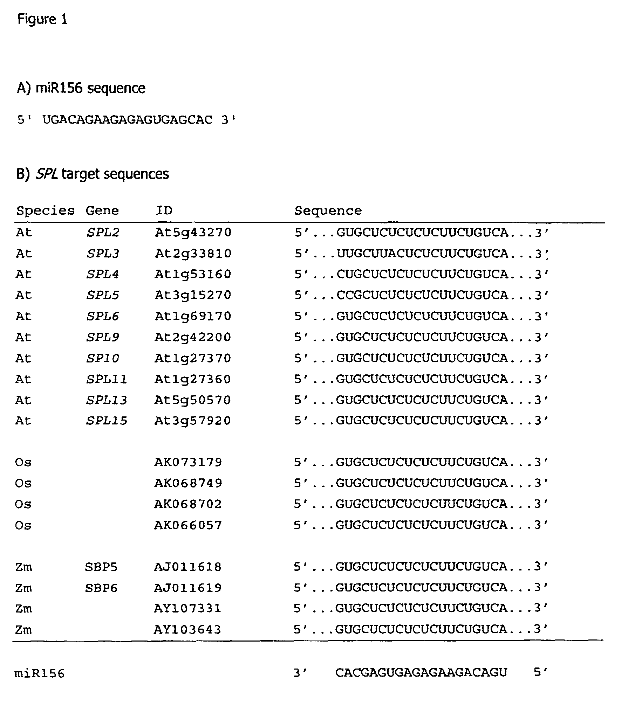

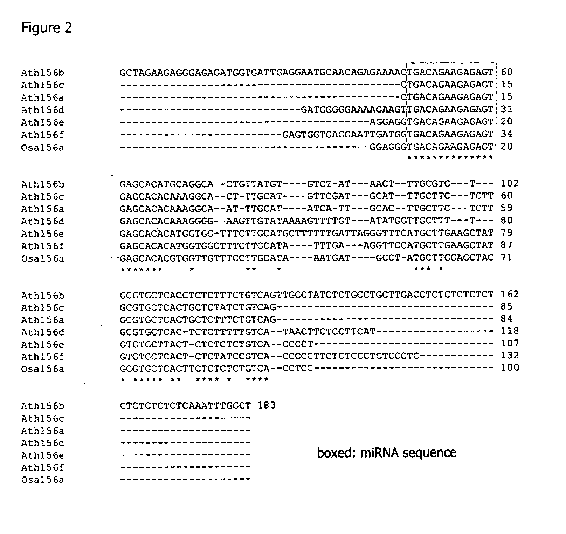

The in vivo functions of most plant miRNAs remain unknown. The microRNA 156 (miR156) of *Arabidopsis thaliana* was first described in Reinhart *et al.* (Genes & Development 16, 1616-26) (Figure 1). The authors cloned the 20 to 21 nucleotide long variants, demonstrated their expression, and also the conservation of miR156 in rice (*Oryza sativa).* There are at least six miR156 precursor genes in *Arabidopsis* (Reinhart *et al.*) and at least 10 in rice, the alignments of these microRNAs being depicted in Figure 2. As predicted by Rhoades *et al.* (Cell 110, 513-20), the potential target genes regulated by miR156 belong to the SPL (*S**QUA* promoter binding protein like) class of transcription factors, which are specific to plants (Cardon *et al,* Gene 237, 91-104).

The function of *SPL* genes with predicted miR156 target sequences is also largely unknown, although many were shown to be upregulated upon flowering (Cardon *et al*; Schmid *et al*), and one of them *SPL3,* has been shown to accelerate flowering when overexpressed in transgenic *Arabidopsis thaliana* plants (Cardon *et al,* Plant Journal 12, 367-377).

There remains a need to develop a method for modifying plant growth characteristics of plants, in particular traits of technical or economical interest such as biomass yield, plant growth rate and plant architecture. Furthermore, there remains a need for transgenic plants with increased biomass yield, growth rate and modified plant architecture.

Accordingly, the problem underlying the present invention is to provide means and methods which enable the modification of biomass yield and/or plant growth and/or plant architecture and particularly, the provision of genetically modified plants which exhibit a modified biomass yield and/or plant growth and/or plant architecture, in particular an increased biomass yield.

This problem is solved by means of the transgenic plants, seed and propagation materials and the methods as defined in the attached claims.

According to a first embodiment, the present invention pertains to a plant with altered expression of at least one microRNA in the plant compared to a wild-type plant. Altered expression is achieved by any of several means, including selection of knockout lines or naturally occurring variants with altered miRNA expression, modification of upstream regulators of miRNA expression, or transgenic constructs that lead to altered expression of the miRNA or miRNA precursor genes. This plant is characterized by exhibiting a modified biomass yield and/or plant growth and/or plant architecture.

According to a second embodiment, the present invention pertains to a plant with altered expression of at least one microRNA that targets members of the *SPL* gene family. Altered expression is achieved by any of several means, including selection of knockout lines or naturally occurring variants with altered miRNA expression, modification of upstream regulators of miRNA expression, or transgenic constructs that lead to altered expression of the miRNA or miRNA precursor genes. This plant is characterized by exhibiting a modified biomass yield and/or plant growth and/or plant architecture.

According to a third embodiment, the present invention pertains to a plant with altered expression of at least one microRNA of the miR156 family in the plant compared to a wild-type plant. Altered expression is achieved by any of several means, including selection of knockout lines or naturally occurring variants with altered miR156 expression, modification of upstream regulators of miR156 expression, or transgenic constructs that lead to altered expression of miR156 or miR156 precursor genes. This plant is characterized by exhibiting a modified biomass yield and/or plant growth and/or plant architecture. Part of the invention is further a plant containing altered levels of a microRNA that has at most three mismatches compared to the miR156 family, which targets members of the *SPL* family of genes encoding SPL transcription factors.

According to a fourth embodiment, the present invention pertains to a transgenic plant that has been genetically modified wherein the genetic modification consists in the introduction of a construct whose presence results in an overexpression of one microRNA of the miR156 family in the plant compared to a wild-type plant. This transgenic plant is characterized by exhibiting a modified biomass yield and/or plant growth and/or plant architecture.

The genetic modification may be any genetic modification leading to an overexpression of microRNAs of the miR156 family in the plant. This encompasses an increased expression as well as a *de novo* expression of microRNAs of the miR156 family. A corresponding genetic modification may be realized through the introduction of a construct comprising a transgene, said transgene comprising genomic fragments of the miRNA156 sequence being expressed in the plant, thus leading to an overexpression of microRNAs of the miR156 family in the plant. Alternatively, the genetic modification may lead to an overexpression of microRNAs of the miR156 family by use of a modulator acting as an enhancer or a suppressor of gene expression, depending on the circumstances (activation, inactivation).

As used herein, the term 'construct' is meant to cover any possible form of genetic information, which provides the information required in the context of the present invention to a particular target cell. The term 'construct' includes any nucleic acid such as DNA, RNA, DNA-RNA-hybrids as well as synthetic analogues thereof such as f.i. PNA.

By 'transgenic' is meant that a plant according to the present invention comprises at least one construct as defined above stably integrated in the genome.

The sequences of the mature miR156 and various microRNAs of the miR156 family are depicted in Figures 1 and 2, respectively. The miRNA156 shown in Figure 1 was isolated from *Arabidopsis* (Reinhart *et al.*). There are at least six miR156 genes in *Arabidopsis* (Reinhart *et al.*), named miRNA 156a, miRNA 156b, miRNA 156c, miRNA 156d, miRNA 156e, and miRNA 156f, and at least 10 in rice; the alignments of these microRNAs being depicted in Figure 2.

The plants according to the present invention may be of any plant species, including monocotyledonous plants or dicotyledonous plants. Examples of monocotyledonous plants include, but are not limited to, asparagus, field and sweet corn, barley, wheat, rice, sorghum, onion, pearl millet, rye and oats, orchids, tulips and lilies. Examples of dicotyledonous plants include, but are not limited to tomato, tobacco, cotton, rapeseed, field beans, soybeans, peppers, lettuce, peas, alfalfa, clover, cabbage crops or *Brassica oleracea (e.g.,* cabbage, broccoli, cauliflower, Brussels sprouts), radish, carrot, beets, eggplant, spinach, cucumber, squash, melons, cantaloupe, sunflowers and various ornamentals. Woody species include poplar, pine, sequoia, cedar, and oak.

Preferably, the method according to the present invention is applied to agricultural useful plants cultivated for food or industrial applications. Accordingly, the present invention pertains to fiber producing plants (e.g. flax, hemp, cotton), oil storing plants (e.g. rape, sunflower, soybean), starch storing plants (e.g. wheat, barley, oats, rye, potato, maize, rice, pea, cassava), sugar storing plants (e.g. sugar beet, sugar-cane) and protein storing plants (e.g. legumes, cereals, soybean). The invention may also be applied to trees and palms. According to a preferred embodiment, the transgenic plants are plants in which the vegetative parts are of interest (crops grown for silage, lettuce and other green vegetables, etc.).

As used herein, the term 'modifying plant biomass yield' means a modified, preferably an increased biomass of either the total plant or of specific parts of the plant such as leaf, shoot, fruit, stem, root, flower, trichome, sepals, hypocotyls, petal, stamen, pollen, style, stigma, seed, embryo, ovule, endosperm, seed coat, nodule, cambium, fiber, aleurone, wood, parenchyma, erenchyma, phloem, sieve element, vascular tissue etc., particularly vegetative parts of plants. Preferably, the biomass of the plant according to the invention is substantially increased compared to wild-type plants by the combined action of significantly higher number of leaves and reduced apical dominance, i.e., an increased number of shoots (branches). Any effect on the biomass yield, compared to the wild type, is part of the present invention. According to preferred embodiments, the biomass yield is increased, favorably substantially increased, and in particular several-fold increased.

As used herein, the term 'plant architecture' denotes the morphology of the plants, and relates to various structural features such e.g. the branching pattern, the number, shape, size, texture, position of organs, cells, and tissues of plants, the traits concerning leaves and flower organs being the major determinants for plant architecture. Examples for plant organs and tissues include leaf, shoot, fruit, stem, root, flower, trichome, sepals, hypocotyls, petal, stamen, pollen, style, stigma, seed, embryo, ovule, endosperm, seed coat, nodule, cambium, fiber, aleurone, wood, parenchyma, erenchyma, phloem, sieve element, vascular tissue etc.

According to a preferred embodiment, the plants according to the present invention show the following traits: the leaf number is increased and/or the apical dominance is reduced and/or the number of shoots is increased. The decreased apical dominance may be manifested in that the main shoot is overgrown by side shoots.

As used herein, the term 'modified plant growth' relates to an alteration of the growth of the plant, in particular of organs and tissues as mentioned in the foregoing. Preferably, it concerns an increase or acceleration of the growth rate of a plant. According to a preferred embodiment, the increase of the leave production rate is in the range of 1.1 to 1.75.

The present invention is further directed to a method for the modification of the biomass yield and/or plant growth and/or plant architecture of a plant, characterized in that the plant is modified in a way leading to an altered expression of microRNAs compared to a wild-type plant. Preferably, it relates to method for the modification of the biomass yield and/or plant growth and/or plant architecture of a plant characterized in that the plant is transformed with a construct leading to an overexpression of a microRNA of the miR156 family in the transgenic plant compared to a wild-type plant. Regarding further specific embodiments of the methods according to the present invention, reference is made to the above-recited annotations regarding transgenic plants according to the present invention.

A further subject of the present invention is transgenic propagation material, seed material, plant parts and transgenic plant cells. In the present context, propagation materials include any part of a plant suitable for vegetative or sexual propagation for the production of progenies, i.e. for example cuttings, callus cultures, rhizomes, tubers, fruits, seeds, seedlings, protoplast etc.

### BRIEF DESCRIPTION OF THE FIGURES

Figure 1A shows the mature RNA sequence of miRNA (isolated from *Arabidopsis thaliana),* according to Reinhart *et al.* (Genes & Development 16, 1616-26). Figure 1B shows an alignment of the miR156 complementary regions of *SPL* mRNAs with miR156. Species are *Arabidopsis thaliana* (At), *Oryza sativa* (Oz, rice) and *Zea mays* (Zm, maize). ID refers to common *Arabidopsis* gene identifiers according to The Arabidopsis Information Resource (http://www.arabidopsis.org) or to GenBank accession numbers.
Figure 2 shows the alignment of the six *Arabidopsis thaliana* miR156 precursors (Ath) and one from rice (Osa).

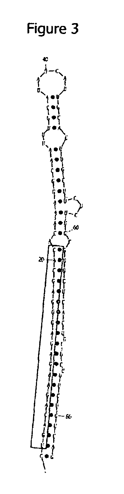

Figure 3 shows the 2D-structure of miR156a fold-back structure as predicted by the mfold algorithm (Zuker, Nucleic Acids Res. 31 (13), 3406-15).

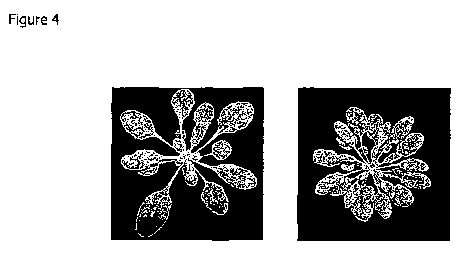

Figure 4 shows transgenic *Arabidopsis thaliana* plants according to the present invention compared to wild-type plants. Left: 33 day-old short-day grown wild-type plant (wt). Right: 35S::miR156c plant, in which the miR156c precursor is expressed from the 35S promoter of cauliflower mosaic virus (Odell *et al,* Nature 313, 810-812).

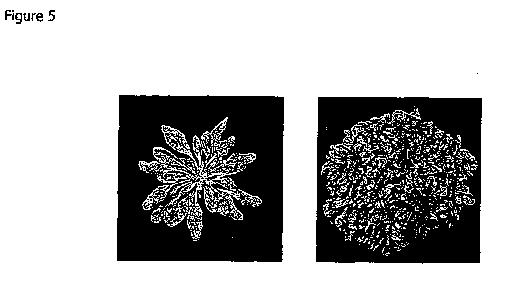

Figure 5 shows transgenic *Arabidopsis thaliana* plants according to the present invention compared to wild-type plants. Left: Short day-grown wild-type plant, which is about to flower. Right: one 35S::miR156f plant, still vegetative but twice the age as wild-type plant. Note the difference in absolute leaf number.

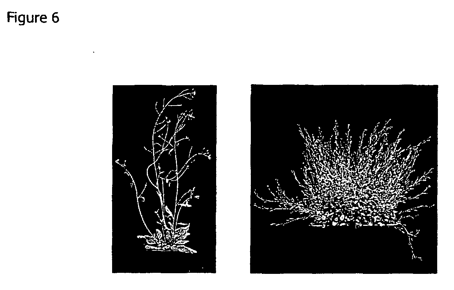

Figure 6 shows transgenic *Arabidopsis thaliana* plants according to the present invention compared to wild-type plants. Left: Long day-grown wild-type plant. Right: 35S::miR156b plants. Note the increased number of shoots.

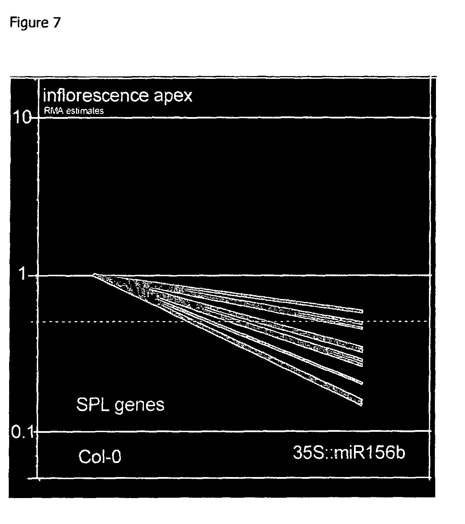

Figure 7 demonstrates decreased expression of *SPL* target genes in plants in which the miR156b precursor is expressed from the 35S promoter of cauliflower mosaic virus. Global gene expression was monitored using Affymetrix GeneChip oligonucleotide arrays (Lockhart *et al*, Nature Biotechnology *14,* 1675-1680). Relative expression levels of 10 *SPL* genes with predicted miR156 target sequences are compared between wild-type (Col-0) and 35S::miR156b plants, with wild-type levels set to 1. Expression levels were estimated using the robust multiarray (RMA) algorithm (Irizarry et al., Nucleic Acids Res. 31, e15). Numbers on the *y* axis indicate relative expression levels.

In the following, the invention will be illustrated by way of non-limiting examples.

### EXAMPLES

In the following examples, miRNAs of the miR156 family were overexpressed in transgenic *Arabidopsis* plants. These plants produced leaves at a rate that was about 1.5 to 2 times faster than control plants, and they had reduced apical dominance, i.e. an increased number of shoots (branches). These two effects combine to increase the biomass substantially.

### Experimental procedure:

Genomic fragments of 1.6 to 2.8 kb in length and centered around the miRNA fold-back sequence (for miRNA156a, b, d and f) as well as a 130 bp genomic fragment containing only the fold-back sequenced (for miRNA156c) were amplified from *Arabidopsis thatiana* genomic DNA of the ecotype Columbia-0 (see Figure 2 and the following description for the miRNA sequences).

The genomic fragments were placed behind the 35S promoter of cauliflower mosaic virus (Odell *et al*) in the pART27 derivative pMLBART (Gleave, Plant Mol. Biol. 20 ,1203 -1207). The resulting constructs were introduced into *Agrobacterium tumefaciens* strains ASE (Fraley *et al,* Biotechnology 3, 629-635). Transgenic *Arabidopsis thaliana* plants of the reference ecotype Columbia-0 (Col-0) were generated using the floral dip method for *Agrobacterium*-mediated transformation (Clough & Bent, Plant Journal 16, 735-743).

In the following, the oligonucleotides used for the amplification of the miR156 genomic fragments are depicted.

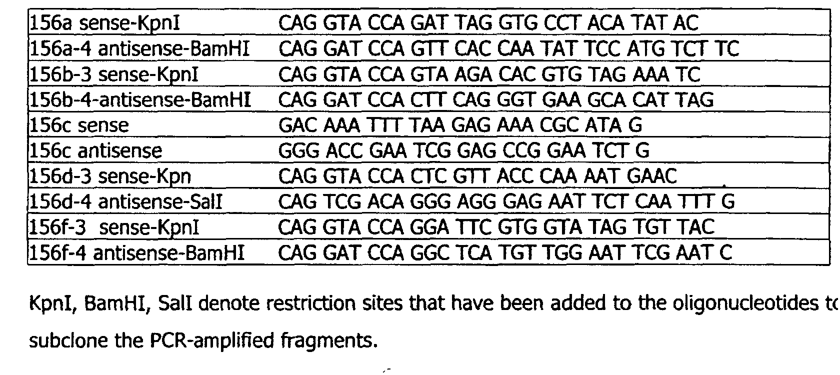

Location of the miRNA genomic fragments that have been overexpressed in plants as annotated by TAIR (www.arabidopsis.org):
156a: chromosome2 - nucleotides 10684693-10682418
156b: chromosome4 - nucleotides 15074536-15076231
156c: chromosome4 - nucleotides 15415545-15415411
156d: chromosome5 - nucleotides 3457848- 3455606
156f: chromosome5 - nucleotides 9134591- 9137382

Seeds from plants infiltrated with the miR156 overexpression constructs were stratified at 4°C for two days, and sown directly on soil. Commercially available Finafe® (AgrEvo), which contains 5.78% of ammonium glufosinate, also known as Basta™, was diluted 1:1000, and herbicide-resistant plants were selected by spraying twice a week for three weeks. Plants were grown either in long days (LD - 16 hours light, 8 hours dark) or short days (SD - 8 hours light, 16 hours dark), at 23°C and about 150 microEinstein per square meter and second light intensity, provided by a 2:1 mixture of Cool White and GroLux (Sylvania) fluorescent lights.

### Rate of leaf initiation in long days and short days

The rosette leaves of transgenic plants according to the present invention were counted at a length of 7mm. Leaf initiation rate (defined as the number of leaves counted from day X to Y divided by (Y-X) days) was determined. 30-40 plants per genotype were investigated. The results are indicated in Tables 1 and 2.

**Table 1**

| Long Day | | | | | | |
|---|---|---|---|---|---|---|
| Genotype | Empty vector (wild-type control, wt) | miR156a over-expression construct | miR156b over-expression construct | miR156d over-expression construct | miR156f over-expression construct | MiR156c over-expression construct |
| Leaf initiation rate | 1.66 | 2.6 | 2.38 | 1.89 | 2.92 | 2.31 |
| Increase relative to wt | (not applicable) | 1.57 | 1.43 | 1.14 | 1.76 | 1.39 |

**Table 2**

| Short Day | | | | | | |
|---|---|---|---|---|---|---|
| Genotype | Empty vector (wild-type control, wt) | miR156a over-expression construct | miR156b over-expression construct | miR156d over-expression construct | miR156f over-expression construct | MiR156c over-expression construct |
| Leaf initiation rate | 1.40 | 2.26 | 2.18 | 1.66 | 2.45 | 1.96 |
| Increase relative to wt | (not applicable) | 1.61 | 1.56 | 1.19 | 1.75 | 1.4 |

The results in Tables 1 and 2 show a substantial increase of leaf initiation rate in miR156 overexpressing plants according to the present invention compared to wild-type plants (empty vector).

### Total number of leaves per plant

All leaves of a plant were counted when the plants started to dry out. 10 plants were investigated for each genotype.

**Table 3**

| Long Day | | | |
|---|---|---|---|
| Genotype | Empty vector (wild-type control, wt) | miR156b overexpression construct | 156d overexpression construct |
| Total leaf number | 127.3 | 1800.5 | 348.5 |
| Increase relative to wt | (not applicable) | 14.14 | 2.74 |

The results in Table 3 (and Figure 4) demonstrate that miR156 overexpressing plants according to the present invention have a substantially higher total number of leaves per plant compared to wild-type plants (empty vector).

### Total number of shoots per plant

When the plants started to dry out, all shoots that had formed fruits (siliques) were counted. 10 plants were investigated for each genotype.

**Table 4**

| Long Day | | | |
|---|---|---|---|
| Genotype | Empty vector (wild-type control, wt) | miR156b overexpression construct | 156d overexpression construct |
| Total shoot number | 28.6 | 313.5 | 82 |
| Increase relative to wt | (not applicable) | 11 | 2.87 |

The results in Table 4 demonstrate that miR156 overexpressing plants according to the present invention have a higher total number of shoots per plant compared to wild-type plants (empty vector).

### Delayed flowering

In both LD and SD conditions, overexpressing miR156 causes late flowering, which often is accompanied by the formation of aerial rosettes (see Figure 5).

### Decreased apical dominance

In both LD and SD conditions, plants overexpressing miR156a, b, c and f show decreased apical dominance: the main shoot is overgrown by side shoots. More than 50% of the primary transformants also start to produce flowers first from side shoots rather than the main shoot (see Figure 6).

## Claims

1. A plant exhibiting modified biomass yield and/or plant growth and/or plant architecture **characterized in that** it contains altered levels of a microRNA.

2. The plant according to claim 1, **characterized in that** it contains altered levels of a microRNA that targets members of the *SPL* family of genes encoding SPL transcription factors.

3. The plant according to claim 1 or 2, **characterized in that** it contains altered levels of a microRNA that is identical to those produced by the miR156 family, which targets members of the *SPL* family of genes encoding SPL transcription factors.

4. The plant according to any of claims 1 to 3, **characterized in that** it contains altered levels of a microRNA that has at most three mismatches compared to the miR156 family, which targets members of the *SPL* family of genes encoding SPL transcription factors.

5. The plant according to any of claims 1 to 4, **characterized in that** it contains altered levels of a microRNA of the miR156 family, which targets members of the *SPL* family of genes encoding SPL transcription factors.

6. A transgenic plant exhibiting modified biomass yield and/or plant growth and/or plant architecture **characterized in that** it has been transformed with a construct leading to an overexpression of microRNAs of the miR156 family in the plant compared to a wild-type plant.

7. The plant according to any of the preceding claims, **characterized in that** production rate of leaves is increased.

8. The plant according to any of the preceding claims, **characterized in that** the number of leaves is increased and/or the apical dominance is reduced.

9. The plant according to any of the preceding claims, **characterized in that** the number of shoots is increased.

10. The plant according to any of the preceding claims, **characterized in that** the biomass yield is increased.

11. The plant according to any of the preceding daims, **characterized in that** the plant shows a delayed flower formation.

12. The transgenic plant according to any of the preceding claims, **characterized in that** the construct comprises a transgene.

13. The transgenic plant according to any of the preceding claims, **characterized in that** the construct comprises a genomic fragment of a microRNA selected from the group consisting of miRNA 156a, miRNA 156b, miRNA 156c, miRNA 156d, miRNA 156e, and miRNA 156f.

14. The transgenic plant according to any of the preceding claims, **characterized in that** the construct comprises a promoter.

15. The transgenic plant according to any of the preceding claims, **characterized in that** the plant is a crop, forage, vegetable, ornamental or woody plant.

16. The transgenic plant according to any of the preceding claims, **characterized in that** the plant is *Arabidopsis thaliana.*

17. Seeds of a transgenic plant according to any of claims 1 to 16.

18. Cells of a transgenic plant according to any of claims 1 to 16.

19. Propagation material of a transgenic plant according to any of claims 1 to 16.

20. A method for the modification of the biomass yield and/or plant growth and/or plant architecture of a plant **characterized in that** the plant is modified, leading to an altered expression of microRNAs compared to a wild-type plant.

21. A method according to claim 20 for the production of a plant according to any of claims 1 to 16.


SEQUENCE LISTING

<110> Max-Planck-Gesell. zur Förd. der Wissenschaften

<120> Means and method for modifying the biomass of plants

<130> EPA-93184

<140> EP04008748.8
<141> 2004-04-13

<160> 36

<170> PatentIn Ver. 2.1

<210> 1
<211> 20
<212> RNA
<213> Arabidopsis thaliana

<220>
<223> miR156 sequence (depicted in Fig. 1A)

<400> 1
ugacagaaga gagugagcac                                                20

<210> 2
<211> 20
<212> RNA
<213> Arabidopsis thaliana

<220>
<223> SPL2 gene (in Fig. 1B)

<400> 2
gugcucucuc ucuucuguca                                                20

<210> 3
<211> 20
<212> RNA
<213> Arabidopsis thaliana

<220>
<223> SPL 3 gene (in Fig. 1B)

<400> 3

uugcuuacuc ucuucuguca                                                    20

<210> 4
<211> 20
<212> RNA
<213> Arabidopsis thaliana

<220>
<223> SPL 4 gene (in Fig. 1B)

<400> 4
cugcucucuc ucuucuguca                                                    20

<210> 5
<211> 20
<212> RNA
<213> Arabidopsis thaliana

<220>
<223> SPL 5 gene (in Fig. 1B)

<400> 5
ccgcucucuc ucuucuguca                                                    20

<210> 6
<211> 20
<212> RNA
<213> Arabidopsis thaliana

<220>
<223> SLP 6 gene (in Fig 1B)

<400> 6
gugcucucuc ucuucuguca                                                    20

<210> 7
<211> 20
<212> RNA
<213> Arabidopsis thaliana

<220>
<223> SPL 9 gene (in Fig 1B)

<400> 7

gugcucucuc ucuucuguca                                                    20

<210> 8
<211> 20
<212> RNA
<213> Arabidopsis thaliana

<220>
<223> SP 10 gene (in Fig 1B)

<400> 8
gugcucucuc ucuucuguca                                                    20

<210> 9
<211> 20
<212> RNA
<213> Arabidopsis thaliana

<220>
<223> SPL 11 gene (in Fig. 1B)

<400> 9
gugcucucuc ucuucuguca                                                    20

<210> 10
<211> 20
<212> RNA
<213> Arabidopsis thaliana

<220>
<223> SPL 13 gene (in Fig. 1B)

<400> 10
gugcucucuc ucuucuguca                                                    20

<210> 11
<211> 20
<212> RNA
<213> Arabidopsis thaliana

<220>
<223> SPL 15 gene (in Fig. 1B)

<400> 11

gugcucucuc ucuucuguca                                                20

<210> 12
<211> 20
<212> RNA
<213> Oryza sativa

<220>
<223> ID AK073179 (in Fig 1B)

<400> 12
gugcucucuc ucuucuguca                                                20

<210> 13
<211> 20
<212> RNA
<213> Oryza sativa

<220>
<223> ID AK068749 (depicted in Fig. 1B)

<400> 13
gugcucucuc ucuucuguca                                                20

<210> 14
<211> 20
<212> RNA
<213> Oryza sativa

<220>
<223> ID AK068702 (in Fig 1B)

<400> 14
gugcucucuc ucuucuguca                                                20

<210> 15
<211> 20
<212> RNA
<213> Oryza sativa

<220>
<223> ID AK066057 (in Fig. 1B)

<400> 15

gugcucucuc ucuucuguca                                                    20

<210> 16
<211> 20
<212> RNA
<213> Zea mays

<220>
<223> SBP5 gene (depicted in Fig. 1B)

<400> 16
gugcucucuc ucuucuguca                                                    20

<210> 17
<211> 20
<212> RNA
<213> Zea mays

<220>
<223> SBP6 gene (depicted in Fig. 1B)

<400> 17
gugcucucuc ucuucuguca                                                    20

<210> 18
<211> 20
<212> RNA
<213> Zea mays

<220>
<223> ID AY107331 (depicted in Fig. 1B)

<400> 18
gugcucucuc ucuucuguca                                                    20

<210> 19
<211> 20
<212> RNA
<213> Zea mays

<220>
<223> ID AY103643 (depicted in Fig. 1B)

<400> 19

gugcucucuc ucuucuguca                                                20

<210> 20
<211> 183
<212> DNA
<213> Arabidopsis thaliana

<220>
<223> alignment to microRNA Ath156b (depicted in Fig. 2)

<400> 20
gctagaagag ggagagatgg tgattgagga atgcaacaga gaaaactgac agaagagagt 60
gagcacatgc aggcactgtt atgtgtctat aactttgcgt gtgcgtgctc acctctcttt 120
ctgtcagttg cctatctctg cctgcttgac ctctctctct ctctctctct ctcaaatttg 180
gct                                                                183

<210> 21
<211> 85
<212> DNA
<213> Arabidopsis thaliana

<220>
<223> alignment to microRNA Ath156c (depicted in Fig. 2)

<400> 21
ctgacagaag agagtgagca cacaaaggca ctttgcatgt tcgatgcatt tgcttctctt 60
gcgtgctcac tgctctatct gtcag                                         85

<210> 22
<211> 84
<212> DNA
<213> Arabidopsis thaliana

<220>
<223> alignment to microRNA Ath156a (depicted in Fig. 2)

<400> 22
ctgacagaag agagtgagca cacaaaggca atttgcatat cattgcactt gcttctcttg 60
cgtgctcact gctctttctg tcag                                          84

<210> 23
<211> 118
<212> DNA
<213> Arabidopsis thaliana

<220>
<223> alignment to microRNA Ath156d (depicted in Fig. 2)

<400> 23
gatgggggaa aagaagttga cagaagagag tgagcacaca aaggggaagt tgtataaaag 60
ttttgtatat ggttgctttt gcgtgctcac tctctttttg tcataacttc tccttcat 118


<210> 24
<211> 107
<212> DNA
<213> Arabidopsis thaliana

<220>
<223> alignment to microRNA Ath156e (depicted in Fig. 2)

<400> 24
aggaggtgac agaagagagt gagcacacat ggtggtttct tgcatgcttt tttgattagg 60
gtttcatgct tgaagctatg tgtgcttact ctctctctgt cacccct 107


<210> 25
<211> 132
<212> DNA
<213> Arabidopsis thaliana

<220>
<223> alignment to microRNA Ath156f (depicted in Fig. 2)

<400> 25
   gagtggtgag gaattgatgg tgacagaaga gagtgagcac acatggtggc tttcttgcat 60
   atttgaaggt tccatgcttg aagctatgtg tgctcactct ctatccgtca cccccttctc 120
   tccctctccc tc 132


<210> 26
<211> 100
<212> DNA
<213> Oryza sativa

<220>
<223> alignment to microRNA Osa156a (depicted in Fig. 2)

<400> 26
ggagggtgac agaagagagt gagcacacgt ggttgtttcc ttgcataaat gatgcctatg 60
cttggagcta cgcgtgctca cttctctctc tgtcacctcc 100

<210> 27
<211> 29
<212> DNA
<213> Arabidopsis thaliana

<220>
<223> 156a sense-KpnI (in exp. part)

<400> 27
caggtaccag attaggtgcc tacatatac                                          29


<210> 28
<211> 32
<212> DNA
<213> Arabidopsis thaliana

<220>
<223> 156a-4 antisense-BamHI (in exp. part)

<400> 28
caggatccag ttcaccaata ttccatgtct tc                                      32


<210> 29
<211> 29
<212> DNA
<213> Arabidopsis thaliana

<220>
<223> 156b-3 sense-KpnI (in exp. part)

<400> 29
caggtaccag taagacacgt gtagaaatc                                          29


<210> 30
<211> 30
<212> DNA
<213> Arabidopsis thaliana

<220>
<223> 156b-4 antisense-BamHI (in exp. part)

<400> 30
caggatccac ttcagggtga agcacattag                                         30

<210> 31
<211> 25
<212> DNA
<213> Arabidopsis thaliana

<220>
<223> 156c sense (in exp. part)

<400> 31
gacaaatttt aagagaaacg catag                                          25


<210> 32
<211> 25
<212> DNA
<213> Arabidopsis thaliana

<220>
<223> 156c antisense (in exp. part)

<400> 32
gggaccgaat cggagccgga atctg                                          25


<210> 33
<211> 28
<212> DNA
<213> Arabidopsis thaliana

<220>
<223> 156d-3 sense-Kpn (in exp. part)

<400> 33
caggtaccac tcgttaccca aaatgaac                                       28


<210> 34
<211> 31
<212> DNA
<213> Arabidopsis thaliana

<220>
<223> 156d-4 antisense-SalI (in exp. part)

<400> 34
cagtcgacag ggagggagaa ttctcaattt g                                   31

<210> 35
<211> 30
<212> DNA
<213> Arabidopsis thaliana

<220>
<223> 156f-3 sense-KpnI (in exp. part)

<400> 35
caggtaccag gattcgtggt atagtgttac                                        30


<210> 36
<211> 31
<212> DNA
<213> Arabidopsis thaliana

<220>
<223> 156f-4 antisense-BamHI (in exp. part)

<400> 36
caggatccag gctcatgttg gaattcgaat c                                      31